# EUROPEAN PATENT APPLICATION

(11) **EP 1 106 177 A1**
(43) Date of publication of application: **13.06.2001**
(21) Application number: 00935661.9
(22) Date of filing: 13.06.2000
(51) Int. Cl.: A61K 31/343, A61K 49/04, A61K 51/04, A61K 47/22, A61P 35/00

(54) **TUMOR TISSUE ACCUMULATION ENHANCERS FOR DRUGS**

(30) Priority: 14.06.1999 JP 16676399
(71) Applicant: TORAY INDUSTRIES, INC., Tokyo 103-8666 (JP); Maeda, Hiroshi, Kumamoto-shi, Kumamoto 862-0909 (JP)
(72) Inventor: MAEDA, Hiroshi, Kumamoto-shi, Kumamoto 862-0909 (JP); HARA, Michio, Kawasaki-shi, Kanagawa 215-0013 (JP)
(74) Representative: Coleiro, Raymond
(86) International application number: JP0003829
(87) International publication number: WO0076496

(57) **Abstract**

An agent for enhancing accumulation of drugs in tumor tissues, which can enhance accumulation of the drugs in tumor tissues, which drugs are used for diagnosis or therapy of tumors, is disclosed. The agent for enhancing accumulation of drugs in tumor tissues according to the invention comprises as an effective ingredient a prostaglandin I derivative such as beraprost or a salt thereof.

## Description

### Technical Field

The present invention relates to an agent for enhancing accumulation of antitumor agents in tumor tissues, which comprises as an effective ingredient a prostaglandin I derivative, which agent is used in combination with the drugs.

### Background Art

It has been confirmed that high molecular substances, fine particles, fats and oils are more accumulated and stay for a longer time in tumor sites and inflammatory sites than in normal tissues. It is thought this is because that leakage of blood components are enhanced due to large production of vascular permeability-increasing factors such as bradykinin, nitrogen monoxide and vascular endothelial cell growth factor in the newly formed blood vessels in tumors, that permeabilities of substances through the blood vessels are higher than those in normal tissues because of the high leakage due to high density of blood vessels, and that absorption by lymphatic system is smaller because lymphatic tissues are lacking (Igakunoayumi, 175, 523, 1995).

It has been tried to effectively transport drugs to tumor cells or tumor tissues so as to promote therapeutic effectiveness exploiting this architectural feature of tumor tissues. For example, there are cases where a protein such as interferon (Cancer and Chemotherapy, 14, 1194, 1987), tumor necrosis factor (Igakunoayumi, 141, 641, 1987) or interleukin (J. Natl. Cancer. Inst., 75, 595, 1985) is used as an antitumor agent, or complexes between a high molecular or fine particulate carrier and an antitumor agent are selectively transported to tumor tissues. As for the transportation to tumor tissues by complexing an antitumor agent with a high molecular substance, known conjugates between an antitumor agent and a high molecular substance include SMANCS (covalently bound conjugate between styrene-maleic acid copolymer and neocarzinostatin)(Gann, 73, 278, 1982), dextran-mitomycin C conjugate (Pharm. Res., 4, 293, 1987), K-18 (covalently bound conjugate between melphalan and IgG) (In ViVo, 1, 205, 1987), Estracyt (conjugate between oestradiol and nitrogen mustards)(Cancer and Chemotherapy, 11, 2106, 1984), conjugate between polyethylene glycol and L-asparaginase (Chem. Lett., 773, 1980), as well as those between an antitumor agent and HPMA polymer, polyvinylpyrrolidone, polyvinyl alcohol or various polyamino acids. As for the transportation of antitumor agents to tumor tissues by making the antitumor agents into fine particles, known such fine particles include cisplatin-encapsulating microspheres, adriamycin-encapsulating albumin microspheres, doxorubicin-encapsulating liposomes (Chem. Rev., 95, 2601, 1995), cisplatin-encapsulating liposomes (J. Neurosurg., 84, 180, 1996) and daunorubicin-encapsulating liposomes (Am. J. Clin. Oncol., 17, 498, 1994). To promote the therapeutic effectiveness of these antitumor agents, combination drugs are also administered. For example, angiotensin II is clinically applied because it selectively increases the blood flow in tumor tissues so as to increase the concentration of the antitumor agent in the tumor tissues (World J. Surg., 19, 836, 1995). The activity of quinine catabolic enzyme inhibitor which is an antihypertensive drug to increase blood vessel permeability of high molecular antitumor agents selectively in tumor tissues is also disclosed (Japanese Laid-open Patent Application (Kokai) No. 1-66130). Further, it has been reported that carboplatin level in uterus tissue was increased by administering prostaglandin E1-containing lipid microspheres before arterial injection of carboplatin against progressive cancer of uterine cervix (Cancer and Chemotherapy, 23, 1697, 1996).

In diagnosis of tumors, imaging by X-ray, MRI or RI scintigraphy is conducted, and contrast media are administered to promote the contrast of tumor images. For example, in X-ray CT diagnosis, various water-soluble and oily iodine-containing contrast media are used. In MRI, gadolinium compounds such as Gd-DTPA (gadolinium chelate of diethylenetriamine pentacetate) are used as contrast media for the diagnosis of tumors (Radiology, 156, 681, 1985). Further, diagnosis of localization and extent of tumors, discrimination of necrosis and recurrence of tumors, evaluation of therapeutic effectiveness and evaluation of malignancy are carried out by administering gallium (⁶⁷Ga) citrate or thallium (²⁰¹Tl) chloride as a contrast medium in RI scintigraphy (Journal of Japan Radiological Society, 51, 415, 1991). In the diagnosis accompanying administration of these contrast media, to promote the clarity and sharpness of tumor images and to decrease the side effects on normal tissues, various trials have been conducted to selectively transport the contrast media to tumor tissues, as in the therapy by antitumor agents. However, sufficient effects have not been obtained.

Prostaglandin I has strong antithrombotic activity and vasodilation activity, and is used for various diseases such as peripheral circulatory disturbance. Some compounds have been found to have anti-ulcer activities, and have various pharmacological activities. However, a case wherein prostaglandin I is used as a combination drug of another drug so as to enhance accumulation of the drug in tumor tissue is not known.

### Disclosure of the Invention

An object of the present invention is to provide an agent for enhancing accumulation of drugs in tumor tissues, which can increase accumulation of the drugs in tumor tissues, which drugs are used for the diagnosis or therapy of tumors.

The present inventors discovered that prostaglandin I derivatives specifically increase permeability of blood vessels in tumors, and decrease the blood flow thereby minimizing the recovery of the drugs from the tumor tissues, so as to enable the drugs dwelling in the tumor tissues at a high concentration, thereby completing the present invention.

That is, the present invention provides an agent for enhancing accumulation of drugs in tumor tissues, comprising as an effective ingredient a prostaglandin I derivative. The present invention also provides a use of the prostaglandin I derivative as an agent for enhancing accumulation of drugs in tumor tissues.

By the present invention, it was confirmed that prostaglandin I derivatives specifically increase permeability of blood vessels in tumors, and decrease the blood flow. By this action, accumulation of the drugs such as antitumor agents and contrast media in tumor tissues can be enhanced, so that therapy and diagnosis of tumors, which are highly safe and effective, are expected to be provided.

### Brief Description of The Drawings

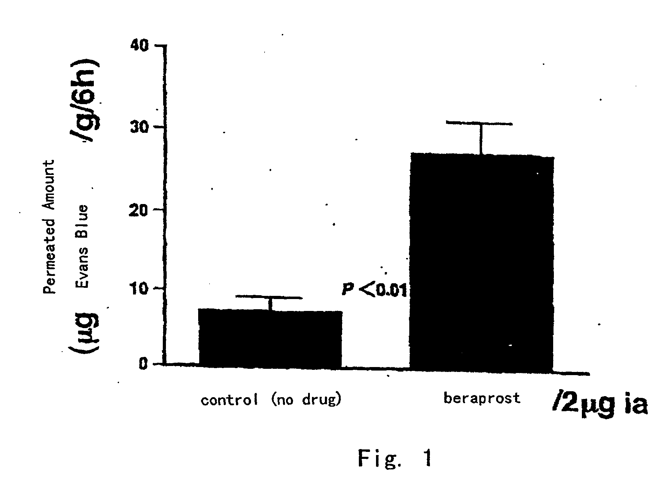

Fig. 1 shows the amounts of permeated Evans Blue in tumor tissues when beraprost sodium was arterially administered and not administered;

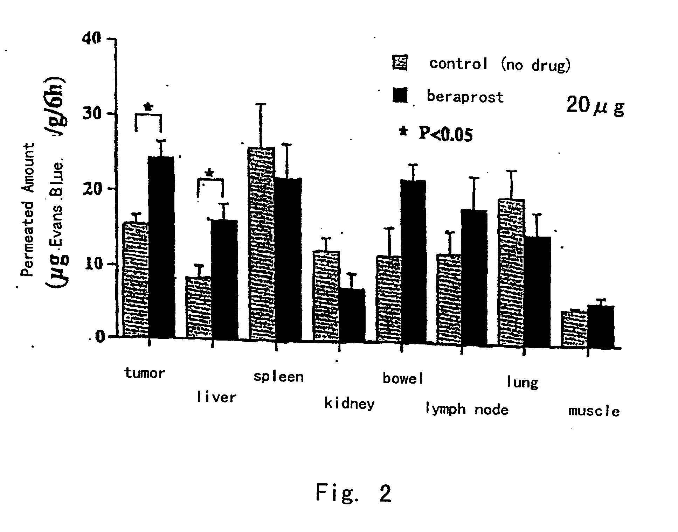

Fig. 2 shows the amounts of permeated Evans Blue in various tissues when beraprost sodium was intravenously administered and not administered;

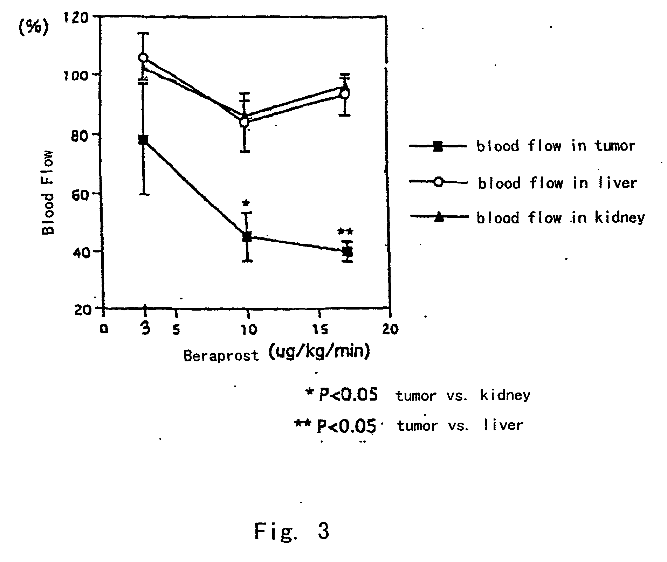

Fig. 3 shows the change in blood flow in various tissues by arterial administration of beraprost sodium; and

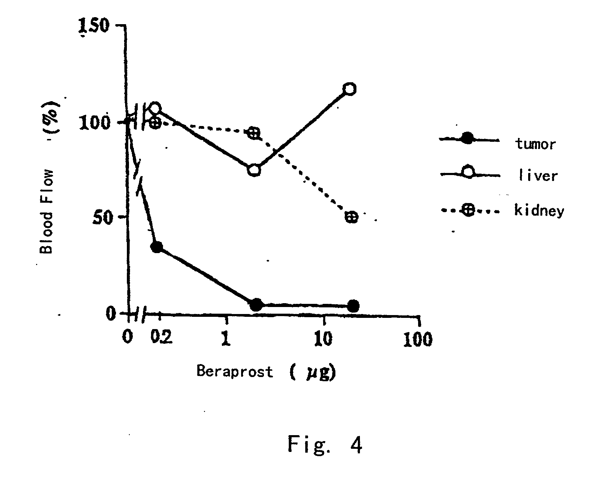

Fig. 4 shows the change in blood flow in various tissues by intravenous administration of beraprost sodium.

### Best Mode for Carrying out the Invention

The drugs to be used in the present invention are not restricted, and examples thereof include antitumor agents and contrast media for the diagnosis of tumors.

The antitumor agents are not restricted as long as they have anti-tumor activities. In view of attaining sufficient therapeutic effects, high molecular antitumor agents and those in the form of fine particles encapsulating effective ingredients therein are preferred. As the high molecular antitumor agents, those having molecular weights of the effective ingredient, or the effective ingredient plus carrier moiety for formulation, if any, of not less than 5000 are preferred. In this case, the effective ingredient *per se* of the antitumor agent may be a high molecular substance. Examples of such an antitumor agent include proteins such as interferon, tumor necrosis factor and interleukin. Alternatively, the effective ingredient may be made into a high molecular substance by complexing with a high molecular carrier. Examples of such an antitumor agent include conjugates between an antitumor agent and a high molecular substance such as SMANCS (covalently bound conjugate between styrene-maleic acid copolymer and neocarzinostatin), dextran-mitomycin C conjugate, K-18 (covalently bound conjugate between melphalan and IgG), Estracyt (conjugate between oestradiol and nitrogen mustards), conjugate between polyethylene glycol and asparaginase, as well as those between an antitumor agent and HPMA polymer, polyvinylpyrrolidone, polyvinyl alcohol or various polyamino acids. On the other hand, as the antitumor agents in the form of fine particles encapsulating effective ingredients therein, those wherein the effective ingredients are encapsulated in liposomes, microspheres, nanospheres, microcapsules, nanocapsules and emulsions are preferred. Among these, those wherein lipids and/or high molecular substances are used as the materials for forming the fine particles are preferred.

The contrast media used for the diagnosis of tumors are not restricted and include oily iodine-containing contrast media such as lipiodol, and water-soluble iodine-containing contrast media such as iopamidol and iohexol for X-ray CT; gadolinium compounds such as Gd-DTPA (gadolinium chelate of diethylenetriamine pentacetate) for the diagnosis of tumors for MRI; and gallium (⁶⁷Ga) chloride- or thallium (²⁰¹TI) citrate-containing injection solutions for RI scintigraphy diagnosis.

Although accumulation in the tumor tissues is observed for any drugs used for the therapy and diagnosis of tumors, this phenomenon is particularly prominent for high molecular drugs and aggregates of molecules such as liposomes. By this phenomenon, more effective and safer diagnosis and therapy of tumors can be attained, and the dose of the drugs can be decreased.

Prostaglandin I derivatives are used as the agent for enhancing accumulation of drugs in tumor tissues. Examples of the prostaglandin I derivatives include prostaglandin I₁ derivatives, prostaglandin I₂ derivatives and prostaglandin I₃ derivatives as well as their salts and esters. Prostaglandin I₂ derivatives as well as their salts and esters may preferably be employed. More preferably, those represented by the following formula (I): 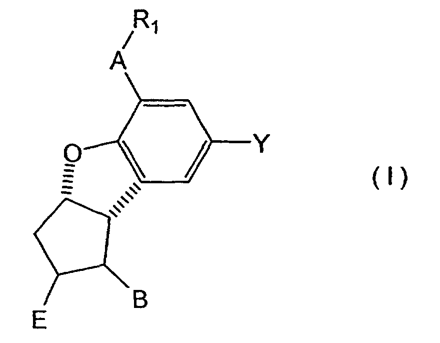 [wherein R₁ represents
(A) COOR₂, wherein R₂ represents
   1) hydrogen or a pharmaceutically acceptable cation,
   2) C₁-C₁₂ straight alkyl or C₃-C₁₄ branched alkyl,
   3) -Z-R₃,
      wherein Z represents covalent bond, or straight or branched alkylene represented by CₜH₂ₜ wherein t represents an integer of 1 to 6, R₃ represents C₃-C₁₂ cycloalkyl or C₃-C₁₂ cycloalkyl substituted with 1 to 3 R₄ wherein R₄ represents hydrogen or C₁-C₅ alkyl,
   4) -(CH₂CH₂O)ₙCH₃
      wherein n represents an integer of 1 to 5,
   5) -Z-Ar₁,
      wherein Z represents the same meanings described above, Ar₁ represents phenyl, α-naphthyl, β-naphthyl, 2-pyridyl, 3-pyridyl, 4-pyridyl, α-furyl, β-furyl, a-thienyl, β-thienyl or substituted phenyl (wherein substituent(s) is(are) at least one chlorine, bromine, fluorine, iodine, trifluoromethyl, C₁-C₄ alkyl, nitro, cyano, methoxy, phenyl, phenoxy, p-acetamide, benzamide, -CH=N-NH-C(=O)-NH₂, -NH-C(=O)-Ph, -NH-C(=O)-CH₃ or -NH-C(=O)-NH₂)
   6) -CₜH₂ₜCOOR₄
      wherein CₜH₂ₜ and R₄ represent the same meanings as described above,
   7) -CₜH₂ₜN(R₄)₂
      wherein CₜH₂ₜ and R₄ represent the same meanings as described above,
   8) -CH(R₅)-C(=O)-R₆
      wherein R₅ represents hydrogen or benzoyl, and R₆ represents phenyl, p-bromophenyl, p-chlorophenyl, p-biphenyl, p-nitrophenyl, p-benzamidephenyl or 2-naphthyl,
   9) -C_{P}H_{2P}-W-R₇
      wherein W represents -CH=CH-, -CH=CR₇- or -C≡C-, wherein R₇ represents hydrogen, C₁-C₃₀ straight or branched alkyl or aralkyl, p represents an integer of 1 to 5, or
   10) -CH(CH₂OR₈)₂
      wherein R₈ represents C₁-C₃₀ alkyl or acyl,
(B) -CH₂OH
(C) -C(=O)N(R₉)₂
   wherein R₉ represents hydrogen, C₁-C₁₂ straight alkyl, C₃-C₁₂ branched alkyl, C₃-C₁₂ cycloalkyl, C₄-C₁₃ cycloalkylalkylene, phenyl, substituted phenyl (wherein the definitions of the substituent(s) are the same as those described in (A)5) mentioned above), C₇-C₁₂ aralkyl or -SO₂R₁₀ wherein R₁₀ represents C₁-C₁₀ alkyl, C₃-C₁₂ cycloalkyl, phenyl, substituted phenyl (wherein the definitions of the substituent are the same as those described in (A)5) mentioned above), or C₇-C₁₂ aralkyl, wherein the two R₉ may be the same or different, with the proviso that when one of them is -SO₂R₁₀, the other R9 is not -SO₂R₁₀, or
(D) -CH₂OTHP (wherein THP represents tetrahydropyranyl),
A represents
1) -(CH₂)ₘ-
2) -CH=CH-CH₂-
3) -CH₂-CH=CH-
4) -CH₂-O-CH₂-
5) -CH=CH-
6) -O-CH₂- or
7) -C≡C-
   wherein m represents an integer of 1 to 3,
Y represents hydrogen, C₁-C₄ alkyl, chlorine, bromine, fluorine, formyl, methoxy or nitro,
B represents -X-C(R₁₁)(R₁₂)OR₁₃
wherein R₁₁ represents hydrogen or C₁-C₄ alkyl, R₁₃ represents hydrogen, C₁-C₁₄ acyl, C₆-C₁₅ aroyl, tetrahydropyranyl, tetrahydrofuranyl, 1-ethoxyethyl or t-butyl,
X represents
1) -CH₂-CH₂-
2) -CH=CH- or
3) -C≡C-
R₁₂ represents
1) C₁-C₁₂ straight alkyl, C₃-C₁₄ branched alkyl, or
2) -Z-Ar₂-
   wherein Z represents the same meanings as described above, Ar₂ represents phenyl, α-naphthyl, β-naphthyl, or phenyl substituted with at least one chlorine, bromine, fluorine, iodine, trifluoromethyl, C₁-C₄ alkyl, nitro, cyano, methoxy, phenyl or phenoxy,
3) -CₜH₂ₜOR₁₄
   wherein CₜH₂ₜ represents the same meanings as described above, R₁₄ represents C₁-C₆ straight alkyl, C₃-C₆ branched alkyl, phenyl, phenyl substituted with at least one chlorine, bromine, fluorine, iodine, trifluoromethyl, C₁-C₄ alkyl, nitro, cyano, methoxy, phenyl or phenoxy, cyclopentyl, cyclohexyl, or cyclopentyl or cyclohexyl substituted with 1 to 4 C₁-C₄ alkyl,
4) -Z-R₃
   wherein Z and R₃ represent the same meanings as mentioned above,
5) -CₜH₂ₜ-CH=C(R₁₅)R₁₆
   wherein CₜH₂ₜ represents the same meanings as mentioned above, R₁₅ and R₁₆ represent hydrogen, methyl, ethyl, propyl or butyl, or
6) -CᵤH₂ᵤ-C≡C-R₁₇
   wherein u represents an integer of 1 to 7, CᵤH₂ᵤ represents straight or branched alkylene, and R₁₇ represents C₁-C₆ straight alkyl,
E represents hydrogen or -OR₁₈
wherein R₁₈ represents C₁-C₁₂ acyl, C₇-C₁₅ aroyl or R₂ (wherein R₂ represents the same meanings as described above),
said formula includes d-isomers, 1-isomers and racemic compounds] are employed. Preferred examples of the "pharmaceutically acceptable cation" in the above-described definitions include alkaline metal ions such as sodium ion and potassium ion.

Specific examples of the prostaglandin I derivatives which may be used in the present invention include beraprost of the formula (II) below, iloprost, clinprost, ataprost, ciprostene, naxaprostene, taprostene, cicaprost, pimilprost,CH-169, SM-10902 and CS570, as well as salts and esters thereof, although the prostaglandin I derivatives are not restricted to these. The salts are not restricted as long as they are pharmaceutically acceptable, and preferred examples include alkaline metal salts such as sodium salt and potassium salt. The esters are also not restricted as long as they are pharmaceutically acceptable. Preferred are the esters mentioned in the definition of R₁ in Formula (I) described above, and especially preferred examples include C₁-C₁₂ straight alkyl esters and C₃-C₁₄ branched alkyl esters.

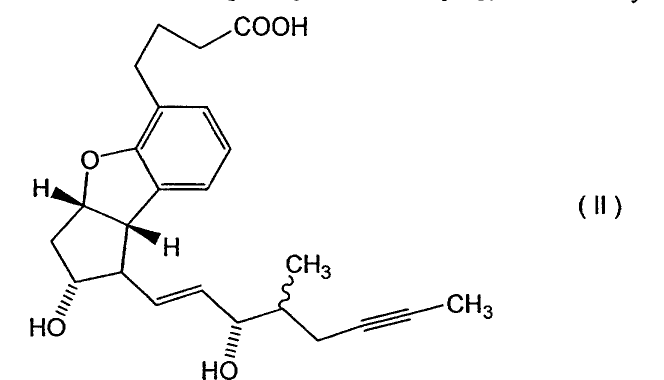

The prostaglandin I derivatives used in the present invention may be produced by known processes. For example, the compounds of the Formula (I) may be produced by the methods described in Japanese Laid-open Patent Application (Kokai) No. 58-124778 and Japanese Patent Publication (Kokoku) No. 6-62594.

The tumors to be treated are not restricted, and examples thereof include malignant tumors in lung, stomach, liver, kidney, cholecyst, pancreas, skin, brain and bladder.

The agent for enhancing accumulation of drugs in tumor tissues may be in the form of tablets, granules, fine granules, capsules, syrups or the like and may be orally administered. Alternatively, it may be administered parenterally by subcutaneous, intramuscular, arterial, intravenous, rectal or percutaneous administration or the like. In formulation for oral administration, formulation techniques such as coating may be employed. In formulation for parenteral administration, carriers for making the drug into fine particles or into high molecular substances may be employed.

The agent for enhancing accumulation of antitumor agents may be administered simultaneously with the antitumor agent, or may be administered at time points different from those at which the drugs such as antitumor agents are administered depending on the purpose of the therapy.

Although the dose of the agent for enhancing accumulation of drugs in tumor tissues differs depending on the type of the tumor, the drug, age and body weight of the patient, the administration method and the like, in case of using beraprost sodium, the dose may be about 0.1 µg to 10 g, preferably 10 µg to 1 g per day per adult.

The present invention will now be described by way of examples thereof. The examples below are presented for the illustration purpose only and should not be interpreted in any restrictive way.

### Example 1

### Permeabilities of Evans Blue in Tumor Tissues When Beraprost Sodium is Arterially Administered and Not Administered

AH136B cells were transplanted to dorsa of feet of Donryu rats to form solid tumors. To each of the tumor-bearing rats, 0.5 mL of solution of Evans Blue (1.5%) in physiological saline was administered *i.v.* and then 2 µg of beraprost sodium was injected into common iliac artery which is the tumor-controlling artery. The blood vessel permeability-increasing effect was evaluated by comparing the amounts of the accumulated Evans Blue-albumin complex in the tissue, which is the index of the blood vessel permeability in the tumor tissue, of the beraprost sodium-administered group and of the non-administered group. As a result, it was observed that by administration of 2 µg of beraprost sodium, the blood vessel permeability was increased to about 3.4 times that of the non-administered group (P<0.01). The amount of the accumulated Evans Blue was spectrophotometrically quantified after extraction with formamide at 30°C for 48 hours.

### Example 2

### Permeabilities of Evans Blue in Various Tissues When Beraprost Sodium is Intravenously Administered and Not Administered

To each of the tumor-bearing rats prepared in the same manner as in Example 1, 0.5 mL of solution of Evans Blue (1.5%) in physiological saline was administered *i.v.* and then 20 µg of beraprost sodium was injected into the tail vein. The blood vessel permeability-increasing effect was evaluated by comparing the amounts of the accumulated Evans Blue-albumin complex in the tissues of the beraprost sodium-administered group and of the non-administered group. As a result, it was observed that by administration of 20 µg of beraprost sodium, the blood vessel permeability was increased to about 1.5 times that of the non-administered group (P<0.05). The amount of the accumulated Evans Blue was quantified in the same manner as in

### Example 1.

### Example 3

### Change in Blood Flow in Various Tissues by Arterial Administration of Beraprost Sodium

Into common iliac artery which is the tumor-controlling artery of the tumor on the dorsum of foot of each of the tumor-bearing rats prepared in the same manner as in Examples 1 and 2, 0.3 mL of beraprost sodium solution (30 µg/mL) in middle-length fatty acid was arterially injected continuously for 1 minute. The changes in the blood flow in various tissues were measured by a Doppler type blood flowmeter. The rate of change was expressed in terms of % of the average during 10 minutes after the beginning of the arterial injection based on the average during several minutes before the arterial injection. The blood flow in the tumor tissue was sharply decreased dose-dependently. This was the phenomenon brought about by the specific relaxation of the blood vessels in the tumor tissues. By the continuous arterial injection of beraprost at a rate of 10 µg/kg/min, the blood flow in the tumor was significantly more decreased than in kidney (P<0.05). By the continuous arterial injection of beraprost at a rate of 17 µg/kg/min, the blood flow in the tumor was significantly more decreased than in kidney and in liver (tumor vs. liver: P<0.05, tumor vs. kidney: P<0.01)

### Example 4

### Change in Blood Flow in Various Tissues by Intravenous Administration of Beraprost Sodium

Into common iliac artery which is the tumor-controlling artery of the tumor on the dorsum of foot of each of the tumor-bearing rats prepared in the same manner as in Examples 1 to 3, 0.2, 2, or 20 µg of beraprost sodium was arterially injected and the change in the blood flow was measured by a Doppler type blood flowmeter. The rate of change was expressed in terms of % of the average during 10 minutes after the beginning of the arterial injection based on the average during several minutes before the arterial injection. Sharp decrease in the blood flow was observed in the tumor. This decrease in the blood flow was not observed in normal tissue organs such as liver and kidney, so that it was a tumor-specific phenomenon.

## Claims

1. An agent for enhancing accumulation of drugs in tumor tissues, comprising as an effective ingredient a prostaglandin I derivative.

2. The agent for enhancing accumulation of drugs in tumor tissues according to claim 1, wherein said prostaglandin I derivative is a prostaglandin I₂ derivative.

3. The agent for enhancing accumulation of drugs in tumor tissues according to claim 1 or 2, wherein said prostaglandin I derivative is represented by the formula (I): 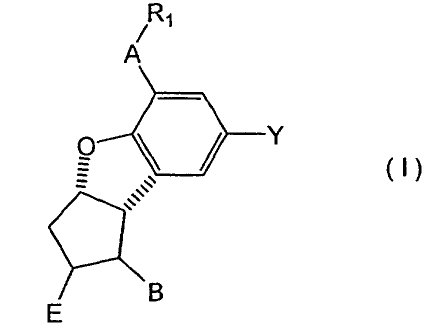 [wherein R₁ represents
(A) COOR₂, wherein R₂ represents
1) hydrogen or a pharmaceutically acceptable cation,
2) C₁-C₁₂ straight alkyl or C₃-C₁₄ branched alkyl,
3) -Z-R₃,
wherein Z represents covalent bond, or straight or branched alkylene represented by CₜH₂ₜ wherein t represents an integer of 1 to 6, R₃ represents C₃-C₁₂ cycloalkyl or C₃-C₁₂ cycloalkyl substituted with 1 to 3 R₄ wherein R₄ represents hydrogen or C₁-C₅ alkyl,
4) -(CH₂CH₂O)ₙCH₃
wherein n represents an integer of 1 to 5,
5) -Z-Ar₁,
wherein Z represents the same meanings described above, Ar₁ represents phenyl, α-naphthyl, β-naphthyl, 2-pyridyl, 3-pyridyl, 4-pyridyl, α-furyl, β-furyl, α-thienyl, β-thienyl or substituted phenyl (wherein substituent(s) is(are) at least one chlorine, bromine, fluorine, iodine, trifluoromethyl, C₁-C₄ alkyl, nitro, cyano, methoxy, phenyl, phenoxy, p-acetamide, benzamide, -CH=N-NH-C(=O)-NH₂, -NH-C(=O)-Ph, -NH-C(=O)-CH₃ or -NH-C(=O)-NH₂)
6) -CₜH₂ₜCOOR₄
wherein CₜH₂ₜ and R₄ represent the same meanings as described above,
7) -CₜH₂ₜN(R₄)₂
wherein CₜH₂ₜ and R₄ represent the same meanings as described above,
8) -CH(R₅)-C(=O)-R₆
wherein R₅ represents hydrogen or benzoyl, and R₆ represents phenyl, p-bromophenyl, p-chlorophenyl, p-biphenyl, p-nitrophenyl, p-benzamidephenyl or 2-naphthyl,
9) -C_{P}H_{2P}-W-R₇
wherein W represents -CH=CH-, -CH=CR₇- or -C≡C-, wherein R₇ represents hydrogen, C₁-C₃₀ straight or branched alkyl or aralkyl, p represents an integer of 1 to 5, or
10) -CH(CH₂OR₈)₂
wherein R₈ represents C₁-C₃₀ alkyl or acyl,
(B) -CH₂OH
(C) -C(=O)N(R₉)₂
wherein R₉ represents hydrogen, C₁-C₁₂ straight alkyl, C₃-C₁₂ branched alkyl, C₃-C₁₂ cycloalkyl, C₄-C₁₃ cycloalkylalkylene, phenyl, substituted phenyl (wherein the definitions of the substituent(s) are the same as those described in (A)5) mentioned above), C₇-C₁₂ aralkyl or -SO₂R₁₀ wherein R₁₀ represents C₁-C₁₀ alkyl, C₃-C₁₂ cycloalkyl, phenyl, substituted phenyl (wherein the definitions of the substituent are the same as those described in (A)5) mentioned above), or C₇-C₁₂ aralkyl, wherein the two R₉ may be the same or different, with the proviso that when one of them is -SO₂R₁₀, the other R₉ is not -SO₂R₁₀, or
(D) -CH₂OTHP (wherein THP represents tetrahydropyranyl), A represents
1) -(CH₂)ₘ-
2) -CH=CH-CH₂-
3) -CH₂-CH=CH-
4) -CH₂-O-CH₂-
5) -CH=CH-
6) -O-CH₂- or
7) -C≡C-
wherein m represents an integer of I to 3,
Y represents hydrogen, C₁-C₄ alkyl, chlorine, bromine, fluorine, formyl, methoxy or nitro,
B represents -X-C(R₁₁)(R₁₂)OR₁₃
wherein R₁₁ represents hydrogen or C₁-C₄ alkyl, R₁₃ represents hydrogen, C₁-C₁₄ acyl, C₆-C₁₅ aroyl, tetrahydropyranyl, tetrahydrofuranyl, 1-ethoxyethyl or t-butyl,
X represents
1) -CH₂-CH₂-
2) -CH=CH- or
3) -C≡C-
R₁₂ represents
1) C₁-C₁₂ straight alkyl, C₃-C₁₄ branched alkyl, or
2) -Z-Ar₂-
wherein Z represents the same meanings as described above, Ar₂ represents phenyl, α-naphthyl, β-naphthyl, or phenyl substituted with at least one chlorine, bromine, fluorine, iodine, trifluoromethyl, C₁-C₄ alkyl, nitro, cyano, methoxy, phenyl or phenoxy,
3) -CₜH₂ₜOR₁₄
wherein CₜH₂ₜ represents the same meanings as described above, R₁₄ represents C₁-C₆ straight alkyl, C₃-C₆ branched alkyl, phenyl, phenyl substituted with at least one chlorine, bromine, fluorine, iodine, trifluoromethyl, C₁-C₄ alkyl, nitro, cyano, methoxy, phenyl or phenoxy, cyclopentyl, cyclohexyl, or cyclopentyl or cyclohexyl substituted with 1 to 4 C₁-C₄ alkyl,
4) -Z-R₃
wherein Z and R₃ represent the same meanings as mentioned above,
5) -CₜH₂ₜ-CH=C(R₁₅)R₁₆
wherein CₜH₂ₜ represents the same meanings as mentioned above, R₁₅ and R₁₆ represent hydrogen, methyl, ethyl, propyl or butyl, or
6) -CᵤH₂ᵤ-C≡C-R₁₇
wherein u represents an integer of 1 to 7, CᵤH₂ᵤ represents straight or branched alkylene, and R₁₇ represents C₁-C₆ straight alkyl,
E represents hydrogen or -OR₁₈
wherein R₁₈ represents C₁-C₁₂ acyl, C₇-C₁₅ aroyl or R₂ (wherein R₂ represents the same meanings as described above),
said formula includes d-isomers, I-isomers and racemic compounds].

4. The agent for enhancing accumulation of drugs in tumor tissues according to claim 3, which is beraprost or a salt thereof.

5. The agent for enhancing accumulation of drugs in tumor tissues according to any one of claims 1 to 4, wherein said drug is an antitumor agent or a contrast medium.

6. The agent for enhancing accumulation of drugs in tumor tissues according to any one of claims I to 5, wherein said drug is a high molecular drug.

7. The agent for enhancing accumulation of drugs in tumor tissues according to any one of claims 1 to 6, wherein effective ingredient of said drug, or complex of effective ingredient moiety and carrier moiety for formulation, has a molecular weight of not less than 5000.

8. Use of said prostaglandin I derivative according to any one of claims 1 to 4 as an agent for enhancing accumulation of drugs in tumor tissues.

9. The use according to claim 8, wherein said drug is an antitumor agent or a contrast medium.

10. The use according to claim 8 or 9, wherein said drug is a high molecular drug.

11. The use according to any one of claims 8 to 10, wherein effective ingredient of said drug, or complex of effective ingredient moiety and carrier moiety for formulation, has a molecular weight of not less than 5000.
